# EUROPEAN PATENT SPECIFICATION

(11) **EP 2 529 057 B1**
(45) Date of publication and mention of the grant of the patent: **13.07.2016**
(21) Application number: 10810968.7
(22) Date of filing: 15.12.2010
(51) Int. Cl.: E03C 1/28, E03C 1/126

(54) **ASSEMBLY FOR DISINFECTING WATER FOR SANITARY FITTINGS**
ANORDNUNG ZUR DESINFEKTION VON WASSER FÜR SANITÄRARMATUREN
ENSEMBLE DE DESINFECTION D'EAU POUR EQUIPEMENTS SANITAIRES

(30) Priority: 29.01.2010 IT RM20100033
(43) Date of publication of application: 05.12.2012
(73) Proprietor: Vitali, Gastone, Civita Castellana (IT)
(72) Inventor: Vitali, Gastone, Civita Castellana (IT)
(74) Representative: Garavelli, Paolo
(86) International application number: PCT/IB2010/003257
(87) International publication number: WO 2011/092544

(56) References cited:
- WO-A1-00/53857
- WO-A1-02/081829
- DE-A1- 4 224 445
- DE-U1- 29 807 712
- FR-A1- 2 902 120
- JP-A- 9 151 505
- KR-A- 20080 086 334
- US-A- 5 891 329

## Description

### TECHNICAL FIELD

The present invention relates to an assembly for disinfecting water for sanitary fittings.

In particular, the present invention regards a sterilizing draining assembly to which the ensuing description will make explicit reference without this implying any loss in generality.

### BACKGROUND ART

In many environments, in particular those in which sanitary activities are carried on, the problem of environmental disinfection is particularly felt on account of the obvious problems that infections may cause. For this purpose considerable economic resources are constantly invested to be able to guarantee an increasingly higher level of sterilization.

In DE29807712U1 a sterilizing siphon according to the preamble of claim 1 is disclosed.

One of the possible causes of infections can derive from the return of viruses and bacteria from the sewage piping to the drain of the sanitary fitting.

Another of the possible causes of infections may regard stagnation of water at the spray heads of showers and in the delivery terminals. In these areas, for example, the *legionella* bacterium finds an environment favourable for its proliferation.

### DISCLOSURE OF INVENTION

The aim of the present invention is to provide an assembly for disinfecting water for sanitary fittings the technical characteristics of which are such as to prevent possible causes of infections.

The subject of the present invention is an assembly for disinfecting water for sanitary fittings the basic characteristics of which are specified in Claim 1 and preferred and/or auxiliary characteristics of which are specified in Claims 2-7.

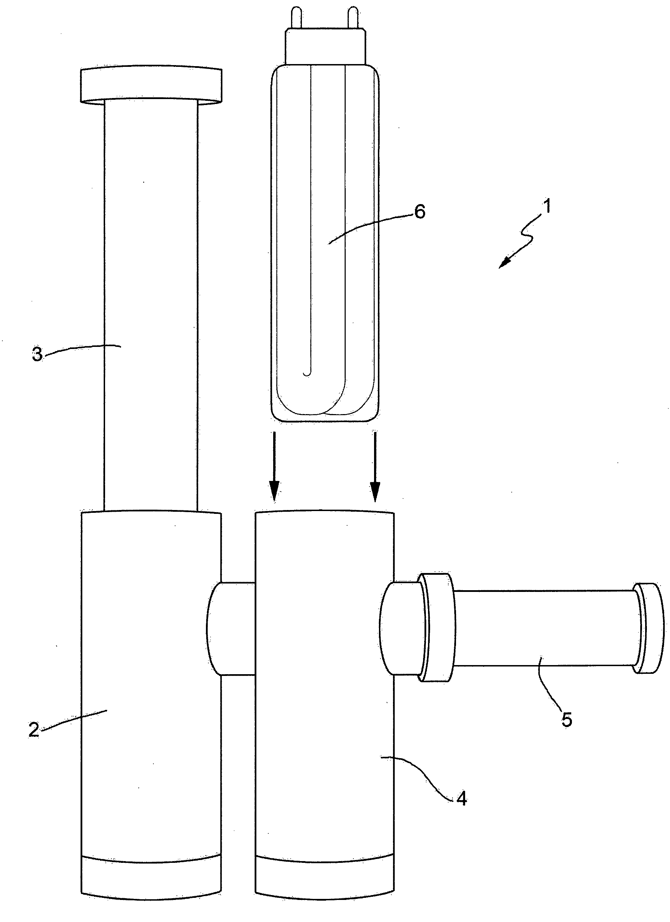

### BRIEF DESCRIPTION OF THE DRAWING

For a better understanding of the invention, embodiments are provided hereinafter purely by way of illustrative and nonlimiting example with the aid of the figure of the annexed drawing, which is a side view of the disinfection assembly according to the present invention with parts separated.

### BEST MODE FOR CURRYING OUT THE INVENTION

In the figure designated as a whole by 1 is a disinfection assembly according to the present invention.

The disinfection assembly 1 comprises a service siphon 2, a first pipe 3 set vertically and designed to connect a drain of a sanitary fitting (known and not illustrated for reasons of simplicity) to the siphon 2, a sterilizing siphon 4 connected to the outlet of the siphon 2, and a second pipe 5 connected to the sterilizing siphon 4 for conveying the waste water towards a sewage system.

The sterilizing siphon 4 comprises a UV lamp 6 immersed in the waste water, which has a bactericidal action and is arranged to irradiate an internal portion of the sterilizing siphon 4 itself in such a way as to ensure that the bactericidal action of the UV radiation takes place in the water contained therein.

In particular, the UV lamp 6 is a low-pressure lamp in such a way as to increase the effectiveness of the bactericidal action up to a percentage of success of 99.9%.

As is known, when a microorganism is exposed to UV radiation, the nuclei of the cells undergo modifications due to photolytic processes. Said modifications lead to blocking of cell division and reproduction with consequent elimination of the micro-organism.

Both of the pipes 3 and 5 are lined with a layer of silicone, which hence will be in direct contact with the waste water.

The silicone layer has the function of hindering bacterial proliferation, thus providing a further barrier to the return of the micro-organisms from the sewage system to the drain.

In particular, the silicone layer is made entirely of dimethyl siloxane.

Also the siphons 2 comprise a silicone lining as described for the pipes 3 and 5. In particular, all the internal walls of the siphon which in use are in contact with the water are lined with a layer of silicone.

In addition, the presence of the double siphon in series renders return of water to the drain of the sanitary fitting to which the draining assembly 1 is applied extremely difficult.

A embodiment of the disinfection assembly not part of the present invention finds advantageous application when applied to a shower spray head. In this case, the UV lamp is inserted within a terminal element, made preferably of steel and set directly upstream of the shower spray head. In this way, the UV lamp of the type described above is arranged so as to irradiate all the water that traverses the terminal element to guarantee a germicidal effect on the water itself.

As has been said above as regards the siphons of the draining assembly, also in this case the internal walls of the terminal element, which in use are in contact with the water, are lined with a layer of silicone.

In this way, in addition to preventing return into the drain of infectious agents coming from the sewage system, sterilization of the water supplied by the sanitary fitting is also guaranteed.

As emerges from the foregoing description, the disinfection assembly forming the subject of the present invention presents technical characteristics such as to guarantee that micro-organisms of infectious agents will be unable to proliferate so that they cannot return from the sewage systems to the drains of the sanitary fittings. In this way, any possible cause of infections is prevented. Obviously, the present invention can find interesting use above all in structures where the environment is more at risk of infections, such as for example hospitals, surgeries, and laboratories.

## Claims

1. An assembly (1) for disinfecting water for sanitary fittings, comprising at least one sterilization siphon (4), which is comprised in an draining assembly and within which water to be disinfected is present; said disinfection assembly being **characterized in that** it comprises a UV lamp (6), housed inside said sterilization siphon (4), immersed in the waste water, and designed to irradiate the water present inside said sterilization siphon (4); said draining assembly comprising a second siphon (2), a first pipe (3) designed to connect a drain of a sanitary fitting to said second siphon (2), and a second pipe (5) designed to drain the waste water from said siphon (2) to a sewage system; said sterilization siphon (4) being set between said second siphon (2) and said second pipe (5).

2. The disinfection assembly according to Claim 1, **characterized in that** said UV lamp (6) emits in a range of wavelengths comprised between 240 and 280 nm.

3. The disinfection assembly according to Claim 1, **characterized in that** said UV lamp (6) emits at a radiating intensity comprised between 9 and 13 mW/cm².

4. The disinfection assembly according to Claim 3, **characterized in that** said UV lamp (6) is a low-pressure lamp.

5. The disinfection assembly according to Claim 1, **characterized in that** said pipes (3, 5) and said siphons (2, 4) comprise a lining layer which is designed to be in contact with the waste water and is made of silicone.

6. The disinfection assembly according to Claim 5, **characterized in that** said lining layer is made entirely of dimethyl siloxane.

7. A sanitary fitting, **characterized in that** it comprises a disinfection assembly according to Claim 1.

## Patentansprüche

1. Gruppe (1) für die Desinfektion von Wasser für Brauchwasseranschlüsse, die mindestens einen Desinfektionsanschluss (4) einschließt, der in einer Drainagegruppe enthalten ist, und in dem das zu desinfizierende Wasser enthalten ist; die genannte Desinfektionsgruppe ist **dadurch gekennzeichnet, dass** sie eine UV-Lampe (6) enthält, die im genannten Desinfektionsanschluss (4) enthalten und in das Abwasser eingetaucht ist, welche entwickelt wurde, um das im genannten Desinfektionsanschluss (4) enthaltene Wasser zu bestrahlen; die genannte Drainagegruppe schließt einen zweiten Anschluss (2), ein erstes Rohr (3), das entwickelt wurde, um einen Abfluss eines Brauchwasseranschlusses mit dem genannten zweiten Anschluss (2) zu verbinden, sowie ein zweites Rohr (5), das entwickelt wurde, um das Abwasser aus dem genannten Anschluss (2) an ein Abwassersystem abzuleiten, ein; der genannte Desinfektionsanschluss (4) ist zwischen dem genannten zweiten Anschluss (2) und dem genannten zweiten Rohr (5) angebracht.

2. Desinfektionsgruppe gemäß Patentanspruch 1, die **dadurch gekennzeichnet ist, dass** die genannte UV-Lampe (6) Licht in einem Wellenlängenbereich zwischen 240 und 280 Nm abgibt.

3. Desinfektionsgruppe gemäß Patentanspruch 1, die **dadurch gekennzeichnet ist, dass** die genannte UV-Lampe (6) eine Strahlungsintensität von 9 bis 13 mW/cm² abgibt.

4. Desinfektionsgruppe gemäß Patentanspruch 3, die **dadurch gekennzeichnet ist, dass** die genannte UV-Lampe (6) eine Niederdrucklampe ist.

5. Desinfektionsgruppe gemäß Patentanspruch 1, die **dadurch gekennzeichnet ist, dass** die genannten Rohre (3, 5) und die genannten Anschlüsse (2, 4) eine Beschichtung enthalten, die dazu entwickelt wurde, mit dem Abwasser in Kontakt zu stehen, und die aus Silikon hergestellt ist.

6. Desinfektionsgruppe gemäß Patentanspruch 5, die **dadurch gekennzeichnet ist, dass** die genannte Beschichtung vollständig aus Dimethylsiloxan hergestellt ist.

7. Brauchwasseranschluss, der **dadurch gekennzeichnet ist, dass** er eine Desinfektionsgruppe gemäß Patentanspruch 1 enthält.

## Revendications

1. Groupe (1) pour désinfecter l'eau pour raccords sanitaires, comprenant au moins un siphon de stérilisation (4), qui est compris dans un groupe de drainage et à l'intérieur duquel se trouve l'eau à désinfecter ; le groupe de désinfection est **caractérisé en ce qu'**il comprend une lampe UV (6), placée à l'intérieur du siphon de stérilisation (4), plongée dans l'eau usée, et projetée pour irradier l'eau contenue dans le siphon de stérilisation (4) ; le groupe de drainage comprend un second siphon (2), un premier tube (3) conçu pour relier l'évacuation d'un raccord sanitaire au second siphon (2), et un second tube (5) conçu pour drainer l'eau usée à partir de ce siphon (2) vers un système d'égout ; le siphon de stérilisation (4) est placé entre le second siphon (2) et le second tube (5).

2. Groupe de désinfection, selon la revendication 1, **caractérisé en ce que** la lampe UV (6) émet dans une gamme de longueurs d'ondes comprise entre 240 et 280 nm.

3. Groupe de désinfection, selon la revendication 1, **caractérisé en ce que** la lampe UV (6) émet à une intensité radiante comprise entre 9 et 13 mW/cm².

4. Groupe de désinfection, selon la revendication 3, **caractérisé en ce que** la lampe UV (6) est une lampe à basse pression.

5. Groupe de désinfection, selon la revendication 1, **caractérisé en ce que** les tubes (3, 5) et les siphons (2, 4) comprennent une couche de revêtement conçue pour entrer en contact avec l'eau, et constituée de silicone.

6. Groupe de désinfection, selon la revendication 5, **caractérisé en ce que** la couche de revêtement est constituée entièrement de diméthylsiloxane.

7. Raccord sanitaire **caractérisé en ce qu'**il comprend un groupe de désinfection selon la revendication 1.
